# EUROPÄISCHE PATENTSCHRIFT

(11) **EP 1 439 170 B1**
(45) Veröffentlichungstag und Bekanntmachung des Hinweises auf die Patenterteilung: **03.06.2009**
(21) Anmeldenummer: 03029973.9
(22) Anmeldetag: 30.12.2003
(51) Int. Cl.: C07D 209/74, C07B 39/00

(54) **Fluorierungsmittel und verfahren zu deren herstellung**
Fluorinating reactants and process for their preparation
Réactifs de fluoration et procédé pour leur préparation

(30) Priorität: 07.01.2003 DE 10300113
(43) Veröffentlichungstag der Anmeldung: 21.07.2004
(73) Patentinhaber: Saltigo GmbH, 40764 Langenfeld (DE)
(72) Erfinder: Ebenbeck, Wolfgang, Dr., 51373 Leverkusen (DE); Marhold, Albrecht, Dr., 51373 Leverkusen (DE); Kolomeitsev, Alexander, Dr., 28205 Bremen (DE); Röschenthaler, Gerd-Volker, Dr., 28357 Bremen (DE)
(74) Vertreter: Wichmann, Birgid

(56) Entgegenhaltungen:
- EP-A- 0 895 991
- WO-A-03/020685
- US-A- 3 092 637
- BRAUER, D. J. ET AL: "Synthesis, vibrational spectra, and crystal structure analysis of di- and trifluoro-tetramethylammonium salts" ZEITSCHRIFT FUER ANORGANISCHE UND ALLGEMEINE CHEMIE , 537, 63-78 CODEN: ZAACAB; ISSN: 0044-2313, 1986, XP0008028797
- FAWCETT F.S. ET AL.: "The chemistry of carbonyl fluoride. I. The fluorination of organic compounds" JOURNAL OF THE AMERICAN CHEMICAL SOCIETY., Bd. 84, Nr. 22, 1962, Seiten 4275-4285, XP002274553 USAMERICAN CHEMICAL SOCIETY, WASHINGTON, DC.
- PATENT ABSTRACTS OF JAPAN Bd. 0101, Nr. 12 (C-342), 25. April 1986 (1986-04-25) & JP 60 243039 A (TOKUYAMA SODA KK), 3. Dezember 1985 (1985-12-03)
- CHEMICAL PHYSICS LETTERS, Bd. 239, Nr. 4, 1995, Seiten 320-325, XP0001189055 NLNORTH-HOLLAND, AMSTERDAM
- KNUNYANTS I. L. ET AL.: "Alpha-fluoroalkylamines, a new source of unhydrated fluoride ion" BULLETIN OF THE ACADEMY OF SCIENCES OF THE USSR. DIVISION OF CHEMICAL SCIENCE., Bd. 30, Nr. 4, 10. Oktober 1981 (1981-10-10), Seiten 639-642, XP002274542 USCONSULTANTS BUREAU. NEW YORK.

## Beschreibung

Die vorliegende Erfindung betrifft ein Verfahren zur Herstellung von α,α-Difluoraminen, Difluormethylen-α,α-diazoverbindungen und Fluorierungsreagenzien enthaltend α,α-Difluoramine und/oder Difluormethylen-α,α-diazoverbindungen.

Verfahren zur Herstellung von α,α-Difluoraminen durch Umsetzung von Dialkyl- bzw. Alkylarylformamide mit Difluorphosgen sind bekannt aus (Z. anorg. Allg. Chem. 1986, 537, 63-78: US 3092637 und J.Am. Chem. Soc., 1962, 84, 4275).

Als Reagenzien zur Fluorierung von Alkoholen oder Carbonylverbindungen wie insbesondere Ketonen, Carbonsäuren und Aldehyden haben sich insbesondere α,α-Difluaramine und Difluormethylen-α,α-diazoverbindungen als besonders geeignet erwiesen. So ist beispielsweise bekannt, zur Fluorierung von sekundären Alkoholen und Carbonsäuren N,N-Dimethyl-1,1-difluorbenzylamin einzusetzen. Allerdings ist für dessen Herstellung der Einsatz des toxischen Schwefeltetrafluorids notwendig (J. Fluoride Chem. 1983, 23, 219-228).

Aus EP-A 895 991 und EP-A 1 013 629 sind Difluormethyten-α,α-diazoverhindungen, wie insbesondere 2,2-Difluor-1,3-dimethylimidazolidin bekannt, die zur fluorierung von Hydroxy- und Carboxylfunktionen eingesetzt werden können. Die Herstellung erfolgt üblicherweise in einer zweistufigen Reaktion, wobei zunächst die entsprechenden Harnstoffderivate mit einem Chlorierungsmittel und anschließend mit ionischem Fluorid zu den gewünschten Produkten umgesetzt werden. Nachteilig sind allerdings die nur mäßigen Ausbeuten. So läuft beispielsweise der Chlor-Fluoraustausch lediglich mit einer Ausbeute von 77 % der Theorie (EP-A 1013 629, S. 145 Beispiel 1).

Es bestand daher das Bedürfnis, ein Verfahren zur Herstellung von α,α-Difluoraminen und Difluormethylen-α,α-diazoverbindungen bereitzustellen, das ohne besondere sicherheitstechnische Massnahmen und in hohen Ausbeuten durchgeführt werden kann.

Es wurde nun ein Verfahren zur Herstellung von Verbindungen der Formel (I) gefunden, 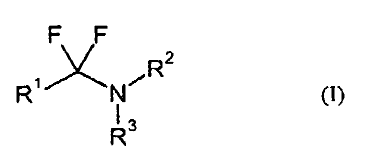 in der
- R¹: für Wasserstoff, C₁-C₁₂-Alkyl, [(C₂-C₁₂-Alkylen)-O]ₙ(C₁-C₁₂-alkyl)] mit n = 1 bis 5, C₃-C₁₄-Aryl, C₄-C₁₅-Arylalkyl oder NR⁴R⁵ steht, wobei R⁴ und R⁵ jeweils unabhängig voneinander für C₁-C₈-Alkyl stehen oder NR⁴R⁵ als Ganzes für einen 4 bis 7 gliedrigen cyclischen Rest mit insgesamt 3 bis 16 Kohlenstoffatomen steht und
- R² und R³: jeweils unabhängig voneinander für C₁-C₁₂-Alkyl, C₃-C₁₄-Aryl oder C₄-C₁₅-Aryl-alkyl stehen oder gemeinsam Teil eines cyclischen Restes mit insgesamt 3 bis 16 Kohlenstoffatomen sind oder
- R¹ und R² und/oder R³: gemeinsam Teil eines cyclischen Restes mit insgesamt 3 bis 16 Kohlenstoffatomen sind,
das dadurch gekennzeichnet ist, dass Verbindungen der Formel (II) 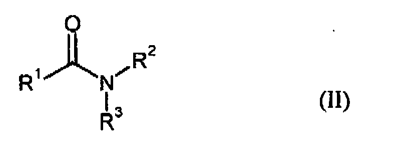 in der R¹, R² und R³ die vorstehend angegebene Bedeutung besitzen
in Gegenwart
- Oxalylfluorid oder
- Oxalylfluorid und Difluorphosgen
und gegebenenfalls organischem Lösungsmittel umgesetzt werden.

Im Rahmen der Erfindung können alle aufgeführten, allgemeinen oder in Vorzugsbereichen genannten Restedefinitionen und Parameter untereinander, also auch zwischen den jeweiligen Bereichen und Vorzugsbereichen in beliebiger Weise kombiniert werden.

Es sei angemerkt, dass die aus Vereinfachungsgründen gewählte, und in der Literatur häufig benutzte Darstellung der Formel (I) auch die nachstehende Darstellung umfasst, die aus eigenen NMR-spektroskopischen Untersuchungen als die wahrscheinlichste Struktur anzunehmen ist

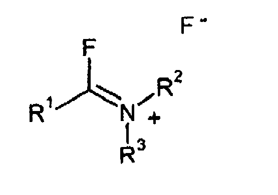

Das Gleiche gilt im Rahmen der Erfindung analog für alle anderen Darstellungen und Nomenklaturen von α,α-Dihalogenoamin-Funktionalitäten.

**Alkyl** beziehungsweise **Alkylen** beziehungsweise **Alkoxy** steht jeweils unabhängig für einen geradkettigen, cyclischen, verzweigten oder unverzweigten Alkyl beziehungsweise Alkylen beziehungsweise Alkoxy-Rest. Gleiches gilt für den nichtaromatischen Teil eines Arylalkyl-Restes.

C₁-C₄-Alkyl steht beispielsweise für Methyl, Ethyl, n-Propyl, iso-Propyl, n-Butyl, sec.-Butyl und tert.-Butyl, C₁-C₈-Alkyl darüber hinaus beispielsweise für n-Pentyl, 1-Methylbutyl, 2-Methylbutyl, 3-Methylbutyl, neo-Pentyl, 1-Ethylpropyl, cyclo-Hexyl, cyclo-Pentyl, n-Hexyl, 1,1-Dimethylpropyl, 1,2-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, 1-Ethyl-2-methylpropyl, n-Heptyl und n-Octyl, C₁-C₁₂-Alkyl weiter darüber hinaus beispielsweise für Adamantyl, die isomeren Menthyle, n-Nonyl, n-Decyl und n-Dodecyl.

C₁-C₄-Alkoxy steht beispielsweise für Methoxy, Ethoxy, n-Propoxy, iso-Propoxy, n-Butoxy, sec.-Butoxy und tert.-Butoxy, C₁-C₈-Alkoxy darüberhinaus für n-Pentoxy, 1-Methylbutoxy, 2-Methylbutoxy, 3-Methylbutoxy, neo-Pentoxy, 1-Ethylpropoxy, cyclo-Hexoxy, cyclo-Pentoxy, n-Hexoxy und n-Octoxy, C₁-C₁₂-Alkoxy weiter darüber hinaus beispielsweise für Adamantoxy, die isomeren Menthoxy-Reste, n-Decoxy und n-Dodecoxy.

C₂-C₁₂-Alkylen steht beispielsweise für 1,2-Ethylen, 1,3-Propylen, 1,4-Butylen, 1,2-cyclo-Hexoxylen und 1,2-cyclo-Pentylen.

**Aryl** steht jeweils unabhängig für einen heteroaromatischen Rest, in dem keines, ein, zwei oder drei Gerüstatome pro Cyclus, im gesamten Molekül mindestens jedoch ein Gerüstatom ausgewählt ist aus der Gruppe Stickstoff, Schwefel oder Sauerstoff oder für einen carbocyclischen aromatischen Rest.

Beispiele für heteroaromatische Reste sind Pyridinyl, Oxazolyl, Benzofuranyl, Dibenzofuran-yl oder Chinolinyl, Beispiele für carbocyclische aromatische Reste Phenyl, Naphtyl, Phenanthrenyl, Anthracenyl oder Fluorenyl.

Weiterhin kann der heteroaromatische oder carbocyclische aromatische Rest mit bis zu fünf gleichen oder verschiedenen Substituenten pro Cyclus substituiert sein, die jeweils unabhängig ausgewählt sind aus der Gruppe Chlor, Fluor, C₁-C₁₂-Alkyl, C₁-C₁₂-Fluoralkyl, C₁-C₁₂-Fluoralkoxy, C₁-C₁₂-Fluoralkylthio, C₁-C₁₂-Alkoxy, Di(C₁-C₈-alkyl)amino oder Tri(C₁-C₈-alkyl)siloxyl.

**Arylalkyl** steht jeweils unabhängig für einen geradkettigen, cyclischen, verzweigten oder unverzweigten Alkyl-Rest nach vorstehender Definition, der einfach, mehrfach oder vollständig durch Aryl-Reste gemäß vorstehender Definition substituiert ist.

Im Folgenden werden die bevorzugten Substitutionsmuster für Verbindungen der Formel (I) definiert:
- R¹: steht bevorzugt für Wasserstoff, C₁-C₁₂-Alkyl, oder C₃-C₆-Aryl, besonders bevorzugt für Wasserstoff oder C₁-C₈-Alkyl und ganz besonders bevorzugt für Wasserstoff oder C₁-C₄-Alkyl.
- R² und R³: stehen bevorzugt jeweils unabhängig voneinander für C₁-C₈-Alkyl oder NR²R³ als Ganzes für N-Morpholinyl, N-Methyl-1,4-Piperazin-N-yl, und besonders bevorzugt jeweils identisch für Methyl, Ethyl oder iso-Propyl.

Ebenfalls bevorzugt steht Formel (I) als Ganzes für 2,2-Difluorimidazolin, 2,2-Difluorpyrrolidin oder 2,2-Difluorpiperidinyl, [2,2,2]-2,2,5,5-Tetrafluor-1,4-diazabicyclooctan oder [2,2,2]-2,2,6,6-Tetraflour-1,4-diazabicyclooctan, wobei die genannten Reste gegebenenfalls einfach oder mehrfach durch C₁-C₄-Alkyl substituiert sein können.

Als Verbindungen der Formel (I) seien genannt:

1,1-Difluormethyl-N,N-dimethylamin, 1,1-Difluormethyl-N,N-diethylamin, 1,1-Difluormethyl-N,N-diisopropylamin, 1,1-Difluor-N,N-2-trimethyl-1-propanamin, 1,1-Difluor-N,N=2,2-tetramethyl-1-propanamin, N,N-Diethyl-α,α-difluor-2,2-dimethyl-1-propanamin, N-(1,1-Difluormethyl)-morpholin, 1,1-Difluor-N,N-dimethylphenylmethanamin, N,N-Diethyl-α,α-difluor-3-pyridylmethanamin, N,N-Diethyl-α,α-difluor-2-pyridylmethanamin, Diethyl-α,α-difluor-(4-chlorphenyl)-methanamin, N,N-Diisopropyl-α,α-difluor-phenylmethanamin, N,N-Diethylyl-α,α-difluor-phenylmethanamin, N,N-Dimethyl-α,α-difluor-phenylmethanamin, 2,2-Difluor-1,3-dimethylimidazolidin und 2,2-Difluor-1,3,3-trimethylpyrrolidin, [2,2,2]-2,2,5,5-Tetrafluor-3,3,6,6-tetramethyl-1,4-diazabicyclooctan und [2.2.2]-2,2,6,6-Tetrafluor-3,3,5,5-tetramethyl-1,4-diazabicyclooctan.

Die Umsetzung von Verbindungen der Formel (I) findet vorzugsweise in Gegenwart von organischem Lösungsmittel statt.

Geeignete organische Lösungsmittel sind beispielsweise aliphatische, alicyclische oder aromatische, gegebenenfalls halogenierte Kohlenwasserstoffe, wie beispielsweise verschiedene Benzine, Benzol, Toluol, Xylol, Chlorbenzol, Dichlorbenzol, verschiedene Petrolether, Hexan, Cyclohexan, Dichlormethan, Chloroform, Tetrachlorkohlenstoff; Ether, wie Diethylether, Methyl-tert.-butylether, Diisopropylether, Dioxan, Tetrahydrofuran oder Ethylenglykoldimethyl- oder -diethylether; Nitrile, wie Acetonitril, Propionitril, Benzonitril, Benzylnitril oder Butyronitril; Sulfone wie Tetramethylensulfon; Benzotrifluorid oder Gemische solcher organischen Lösungsmittel.

Vorzugsweise beträgt der Wassergehalt des Lösungsmittels im erfindungsgemäßen Verfahren maximal 0,2 Gew.-%, bevorzugt maximal 0,05 Gew.-%. Vorzugsweise wird ein solcher Wassergehalt durch Andestillieren oder Trocknung in an sich bekannter Weise erreicht.

Das molare Verhältnis von Oxalylfluorid und/oder Difluorphosgen zu Verbindungen der Formel (I) beträgt beispielsweise und bevorzugt 0,8:1 bis 20:1, bevorzugt 1:1 is 2:1 und besonders bevorzugt 1,02:1 bis 1,1:1. Der Einsatz größerer Mengen ist möglich, bringt jedoch keine Verbesserung der Ausbeute mit sich.

Die Reaktionstemperatur kann beispielsweise -50°C bis 100°C betragen, bevorzugt -10°C bis 50°C.

Der Reaktionsdruck kann beispielsweise 0,8 bis 20 bar betragen, bevorzugt sind 1,5 bis 5 bar.

Die Aufarbeitung nach der Reaktion kann z. B. durch Abdestillieren aller flüchtigen Bestandteile und Trocknen des Rückstandes im Hochvakuum erfolgen.

Auf erfindungsgemäße Weise werden die Verbindungen der Formel (I) in hoher Ausbeute und Reinheit erhalten.

Überraschenderweise wurde gefunden, dass Verbindungen der Formel (I) als Fluorierungsreagenz noch effizienter wirken, wenn sie in Gegenwart eines tertiären aprotischen Amins und/oder einer N-heteroaromatischen Verbindung und in Gegenwart von Fluorwasserstoff eingesetzt werden.

Daher ist von der Erfindung auch ein Verfahren umfasst, dass als weiteren Schritt die Umsetzung der erfindungsgemäß hergestellten Verbindungen der Formel (I) mit
- zumindest einem, bevorzugt genau einem aprotischen, tertiären Amin das in -Stellung zum Stickstoff keine Fluoratome enthält und/oder zumindest einer, bevorzugt genau einer N-heteroaromatischen Verbindung und
- Fluorwasserstoff
umfasst.

Aprotisch bedeutet in diesem Zusammenhang, dass das tertiäre Amin, das auch ein Molekül mit mehreren tertiären Aminogruppen sein kann keine Wasserstoffatome trägt die bezogen auf eine wässrige Vergleichsskala bei 25°C einen pKs-Wert von weniger als 20 aufweisen.

Es sei angemerkt, dass unter den oben aus Vereinfachungsgründen gewählten Begrifflichkeiten auch die entsprechenden tertiären Ammonium-fluoride, N-Heteroaryliumfluoride, und die entsprechenden Polyfluoride sowie die Polyfluoride von ionischen Verbindungen der Formel (I) umfasst sind (siehe Darstellung (Ia)), die bei der Reaktion von tertiären Aminen und/oder N-heteroaromatischen Verbindungen oder Verbindungen der Formel (I) und Fluorwasserstoff auftreten.

Bevorzugte aprotische tertiäre Amine sind solche der Formeln (IIIa) und (IIIb)

NR⁶R⁷R⁸ (IIIa)

(R⁹)N-F-N(R⁹)₂ (IIIb)

in der
- R⁶, R⁷ und R⁸: jeweils unabhängig voneinander für C₁-C₁₂-Alkyl oder [(C₂-C₁₂-Alkylen)-O]ₙ(C₁-C₁₂-alkyl)] mit n = 1 bis 5 stehen oder zwei oder drei der Reste R⁶, R⁷ und/oder R⁸ mit dem Stickstoffatom einen mono- bzw. bi-cyclischen Rest mit insgesamt 3 bis 16 bzw. 5 bis 20 Kohlenstoffatomen bilden und
- R⁹: jeweils unabhängig für C₁-C₁₂-Alkyl steht oder jeweils zwei der vier Reste zusammen für einen C₂-C₈-Alkylen-Rest stehen und
- F: für C₂-C₈-Alkylen steht.

In Formel (IIIa) stehen bevorzugt R⁶, R⁷ und R⁸ jeweils unabhängig voneinander für C₁-C₁₂-Alkyl, in der Formel besonders bevorzugt jeweils identisch für C₁-C₈-Alkyl. Besonders bevorzugte aprotische tertiäre Amine der Formeln (IIIa) und (IIIb) sind Triethylamin, Diazabicyclooctan und Tetramethylethylendiamin.

Bevorzugte N-heteroaromatische Verbindungen sind gegebenenfalls substituierte Pyridine und Chinoline, wobei Pyridin besonders bevorzugt ist.

Das molare Verhältnis von aprotischem tertiären Amin zu Verbindungen der Formel (I) beträgt beispielsweise und bevorzugt 0,1:1 bis 20: 1, bevorzugt 1: 1 bis 10:1.

Das molare Verhältnis von Fluorwasserstoff zu aprotischem tertiärem Amin und/oder N-heteroaromatischen Verbindungen beträgt beispielsweise und bevorzugt 0,2:1 bis 10:1 pro Stickstoffatom.

Für die Umsetzung mit aprotischem tertiären Amin und/oder N-heteroaromatischen Verbindungen und Fluorwasserstoff kann beispielsweise so vorgegangen werden, dass Verbindungen der Formel (I) vorgelegt werden und zunächst mit aprotischem tertiären Amin und/oder N-heteroaromatischen Verbindungen und anschließend mit Fluorwasserstoff oder zunächst mit Fluorwasserstoff und anschließend mit aprotischem tertiären Amin oder N-heteroaromatischen Verbindungen umgesetzt werden, oder vorzugsweise mit Mischungen aus aprotischem tertiärem Amin oder N-heteroaromatischen Verbindungen und Fluorwasserstoff, die in verschiedenen Zusammensetzungen wie z.B. (NEt₃ x 3 HF) oder (Pyridin x 9HF) auch kommerziell erhältlich sind, umgesetzt werden. Auch andere Zugabeabfolgen führen in gleicher Weise zum Erfolg.

Die erfindungsgemäß herstellbaren Verbindungen der Formel (I) oder deren Mischungen mit tertiärem, aprotischen Amin und/oder N-heteroaromatischen Verbindungen und Fluorwasserstoff eignen sich insbesondere zur Herstellung von Fluorverbindungen aus den entsprechenden Hydroxyverbindungen sowie zur Herstellung von geminalen-Difluorverbindungen aus den entsprechenden Carbonylverbindungen. Bevorzugte Fluorverbindungen sind solche, die zur Herstellung von Agrochemikalien, Arzneimitteln und Flüssigkristallen eingesetzt werden.

Das erfindungsgemäße Verfahren besitzt den Vorteil, dass die Verbindungen der Formel (I) oder deren Mischungen mit tertiärem, aprotischen Amin und/oder N-heteroaromatischen Verbindungen und Fluorwasserstoff von leicht erhältlichen Edukten ausgeht und die gewünschten Produkte bei sehr einfacher Aufarbeitung in sehr hohen Reinheiten und Ausbeuten liefert.

### Beispiele:

### Beispiel 1

### Herstellung von 1,1-Difluor-N,N-2,2-tetramethyl-1-propanamin

Unter Schutzgas-Atmosphäre werden in einem Stahlzylinder 2,58 g (20 mmol) N,N-Dimethylpivalamid zusammen mit 10 ml CH₂Cl₂ vorgelegt. Man kühlt den Ansatz auf -10°C ab und dosiert hierzu 2 g (21,3 mmol) vorgekühltes Oxalylfluorid. Das Reaktionsgefäß wird verschlossen, man lässt auf Raumtemperatur kommen und erwärmt den Ansatz unter Rühren für weitere 16 h auf 40°C. Nach Vervollständigung der Reaktion (GC) lässt man wieder auf 20°C abkühlen. Nach Abziehen des Lösungsmittels im Wasserstrahlvakuum erhält man 1,1-Difluor-N,N-2,2-tetramethyl-1-propanamin als leicht gelbe Flüssigkeit.

Ausbeute: 2,85 g (18,5 mmol; 93 %; Reinheit: 99 % (bezogen auf Fläche)

¹H-NMR (CDCl₃): 1,00 (s breit, 9H, *t*-Bu-*H*), 2,26 (t, 6H, *⁴J*_{HF} = 1,95 Hz, N(C*H*₃)₂) ppm.

¹³C-NMR (C₆D₆): 25,7 (s, CH_{3,} 3C, *t*-Bu-CH₃), 38,3 (t, CH_{3,} ³*J*_{CF} = 6,03 Hz, N(CH₃)₂), 40,0 (t, quart. C, 1C, ²*J*_{CF} = 29,8 Hz, *t*-Bu-C), 128,6 (t, CF₂, 1C, ¹*J*_{CF} = 258,1 Hz) ppm.

¹⁹F-NMR (CDCl₃): -97,5 (s, -CF₂) ppm.

### Beispiel 2

### Herstellung eines Fluorierungsreagenzes enthaltend 1,1-Diftuormethyl-N,N-diisopropylamin

Unter Schutzgas-Atmosphäre werden in einem Polyethylen-Kolben 10,4 g (69 mmol) 1,1-Difluormethyl-N,N-diisopropylamin, das analog zu Beispiel 1 in einer Ausbeute von 92 % d. Th. hergestellt wurde, vorgelegt und auf 0°C abgekühlt. Dann werden innerhalb von 2 min 11,1 g (69 mmol) NEt₃•3HF zudosiert und noch 20 min bei dieser Temperatur gerührt. Die zunächst flüssigkristalline Reaktionsmischung lässt man auf 20°C kommen, erwärmt zur Homogenisierung für 0.5 h auf 40°C und lässt wieder auf 20°C erkalten. Hieraus resultieren 21,5 g (69 mmol) ***i*-Prop₂N=CHF⁺HF₂⁻ • HNEt₃⁺• HF₂⁻** mit einem Schmelzpunkt von 37-40°C.

¹⁹F-NMR (CD₂Cl₂): -86.7 (br s, 1F, CHF⁺), -158.5 (br s, 4F, HF₂⁻) ppm.

### Beispiel 3

### Umsetzung von 1-Phenylethanol mit dem Fluorierungsreagenz aus Beispiel 2

In einem PE-Gefäß tropft man unter Schutzgas-Atmosphäre zu einer Lösung aus 2,32 g (7,56 mmol) ***i*-Prop₂N=CHF⁺HF₂⁻• HNEt₃⁺• HF₂⁻** in 10 ml CH₂Cl₂ 0,83 g (6,8 mmol) 1-Phenytethanol innerhalb von 5 min. Man rührt für mehrere Stunden bei 20°C und analysiert den Umsatz durch ¹⁹F-NMR (Referenz: PhCF₃). Nach 2,5 h erhält man 81 % 1-Fluorethylbenzol, nach 24 h Rührzeit erhält man 96 % Produkt.

## Patentansprüche

1. Verfahren zur Herstellung von Verbindungen der Formel (I) 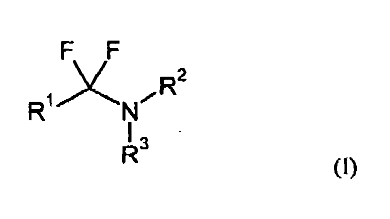 in der
R¹ für Wasserstoff, C₁-C₁₂-Alkyl, [(C₂-C₁₂-Alkylen)-O]ₙ(C₁-C₁₂-alkyl)] mit n = 1 bis 5, C₃-C₁₄-Aryl, C₄-C₁₅-Arylalkyl oder NR⁴R⁵ steht, wobei R⁴ und R⁵ jeweils unabhängig voneinander für C₁-C₈-Alkyl stehen oder NR⁴R⁵ als Ganzes für einen 4 bis 7 gliedrigen cyclischen Rest mit insgesamt 3 bis 16 Kohlenstoffatomen steht und
R² und R³ jeweils unabhängig voneinander für C₁-C₁₂-Alkyl, C₃-C₁₄-Aryl oder C₄-C₁₅-Arylalkyl stehen oder gemeinsam Teil eines cyclischen Restes mit insgesamt 3 bis 16 Kohlenstoffatomen sind oder
R¹ und R² und/oder R³ einen cyclischen Rest mit insgesamt 3 bis 16 Kohlenstoffatomen sind,
**dadurch gekennzeichnet, dass** Verbindungen der Formel (11) 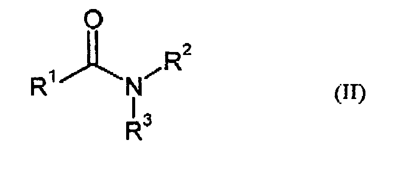 in der
R¹, R² und R³ die vorstehend angegebene Bedeutung besitzen
in Gegenwart von
• Oxalylfluorid oder
• Oxalylfluorid oder
• Oxalylfluorid und Difluorphosgen
umgesetzt werden.

2. Verfahren nach Anspruch 1, **dadurch gekennzeichnet, dass** die Umsetzung in Gegenwart von organischem Lösungsmittel stattfindet.

3. Verfahren nach mindestens einem der Ansprüche 1 und 2, **dadurch gekennzeichnet, dass** R¹ für Wasserstoff, C₁-C₁₂-Alkyl oder C₃-C₆-Aryl steht.

4. Verfahren nach mindestens einem der Ansprüche 1 bis 3, **dadurch gekennzeichnet, dass** R² und R³ jeweils unabhängig voneinander für C₁-C₈-Alkyl oder NR²R³ als Ganzes für N-Morpholinyl, N-Methyl-1,4-Piperazin-N-yl stehen oder R¹CF₂R² als Ganzes für 2,2-Difluorimidazolinyl, 2,2-Difluorpyrrolidinyl, 2,2-Difluorpiperidinyl oder [2.2.2]-2,2,5,5-Tetrafluor-1,4-diazabicyclooctan oder [2,2,2],2,2,6,6-Tetmfluor-1,4-diazabicyclooctan steht, wobei die genannten Reste gegebenenfalls einfach oder zweifach durch C₁-C₄-Alkyl substituiert sein können.

5. Verfahren nach mindestens einem der Ansprüche 1 bis 4, **dadurch gekennzeichnet, dass** als Verbindungen der Formel (1) hergestellt werden: 1,1-Difluormethyl-N,N-dimethylamin, 1,1-Difluormethyl-N,N-diethylamin, 1,1-Difluomethyl-N,N-diisopropylamin, 1,1-Difluor-N,N-2-trimethyl-1-propanamin, 1,1-Difluor-N,N-2,2-tetramethyl-1-propanamin, N,N-Diethyl-α,α-difluor-2,2-dimethyl-1-propanamin, N-(1,1-Difluormethyl)-morpholin, 1,1-Difluor-N,N-dimethylphenylmethanamin, N,N-Diethyl-α,α-difluor-3-pyridylmethanamin, N,N-Diethyl-α,α-difluor-2-pyridylmethanamin, Diethyl-α,α-difluor-(4-chlorphenyl)-methanamin, N,N-Diisopropyl-α,α,difluor-phenylmethanamin, N,N-Diethylyl-α,α-difluorphenylmethanamin, N,N-Dimethyl-α,α-difluor-phenylmethanamin, 2,2-Difluor-1,3-dimethylimidazolidin, 2,2-Difluor-1,3,3-trimethylpyrrolidin, [2,2,2]-2,2,5,5-Tetrafluor-3,3,6,6-tetramethyl-1,4-diazabicyclooctan und [2.2.2]-2,2,6,6-Tetrafluor-3,3,5,5-tetramethyl-1,4-diazabicyclooctan.

6. Verfahren nach mindestens einem der Ansprüche 1 bis 5, **dadurch gekennzeichnet, dass** das molare Verhältnis von Oxalylfluorid zu Verbindungen der formel (I) 0,8:1 bis 20;1 beträgt.

7. Verfahren nach mindestens einem der Ansprüche 1 bis 6, **dadurch gekennzeichnet, dass** die Reaktionstemperatur -50°C bis 100°C beträgt.

8. Verfahren nach mindestens einem der Ansprüche 1 bis 7, **dadurch gekennzeichnet, dass** der Reaktionsdruck 0,8 bis 20 bar beträgt.

9. Verfahren nach mindestens einem der Ansprüche 1 bis 8, **dadurch gekennzeichnet, dass** in einem weiteren Schritt die Umsetzung mit
• zumindest einem aprotischen, tertiären Amin das in α-Stellung zum Stickstoff keine Fluoratome enthält und/oder zumindest einer N-heteroaromatischen Verbindung und
• Fluorwasserstoff
erfolgt.

10. Verfahren nach Anspruch 9, **dadurch gekennzeichnet, dass** das molare Verhältnis von aprotischern tertiärem Amin und/oder N-heteroatomatischen Verbindungen zu Verbindungen der Formel (I) 0,1:1 bis 20:1 und das molare Verhältnis von Fluorwasserstoff zu aprotischem tertiärem Amin 0,2:1 bis 10:1 beträgt.

11. Verfahren zur Herstellung von Fluorverbindungen aus entsprechenden Hydroxyverbindungen sowie zur Herstellung von geminalen-Difluorverbindungen aus den entsprechenden Carbonylverbindungen durch Verbindungen die nach mindestens einem der Ansprüche 1 bis 10 hergestellt wurden..

## Claims

1. Process for preparing compounds of the formula (I) 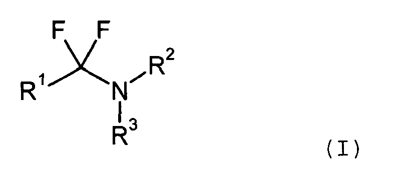 where
R¹ represents hydrogen, C₁-C₁₂-alkyl, [(C₂-C₁₂-alkylene)-O]ₙ(C₁-C₁₂-alkyl) where n = 1 to 5, C₃-C₁₄-aryl, C₄-C₁₅-arylalkyl or NR⁴R⁵, where R⁴ and R⁵ each independently of one another represent C₁-C₈-alkyl or NR⁴R⁵ as a whole represents a 4 to 7-membered cyclic radical having a total of 3 to 16 carbon atoms and
R² and R³ each independently of one another represent C₁-C₁₂-alkyl, C₃-C₁₄-aryl or C₄-C₁₅-arylalkyl, or together are part of a cyclic radical having a total of 3 to 16 carbon atoms, or
R¹ and R² and/or R³ are a cyclic radical having a total of 3 to 16 carbon atoms,
**characterized in that** compounds of the formula (II) 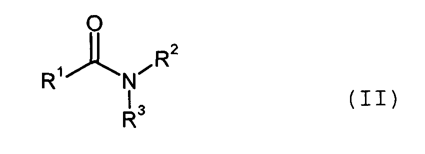 where
R¹, R² and R³ have the meanings given above
are reacted in the presence of
• oxalyl fluoride or
• oxalyl fluoride and difluorophosgene.

2. Process according to Claim 1, **characterized in that** the reaction takes place in the presence of organic solvent.

3. Process according to at least one of Claims 1 and 2, **characterized in that** R¹ represents hydrogen, C₁-C₁₂-alkyl or C₃-C₆-aryl.

4. Process according to at least one of Claims 1 to 3, **characterized in that** R² and R³ each independently of one another represent C₁-C₈-alkyl, or NR²R³, as a whole, represent N-morpholinyl, N-methyl-1,4-piperazin-N-yl, or R¹CF₂R², as a whole, represents 2,2-difluoroimidazolinyl, 2,2-difluoropyrrolidinyl, 2,2-difluoropiperidinyl or [2,2,2]-2,2,5,5-tetrafluoro-1,4-diazabicyclooctane or [2,2,2]-2,2,6,6-tetrafluoro-1,4-diazabicyclooctane, in which case the said residues can, if appropriate, be monosubstituted or disubstituted by C₁-C₄-alkyl.

5. Process according to at least one of Claims 1 to 4, **characterized in that** the compounds of the formula (I) prepared are: 1,1-difluoromethyl-N,N-dimethylamine, 1,1-difluoromethyl-N,N-diethylamine, 1,1-difluoromethyl-N,N-diisopropylamine, 1,1-difluoro-N,N-2-trimethyl-1-propanamine, 1,1-difluoro-N,N-2,2-tetramethyl-1-propanamine, N,N-diethyl-α,α-difluoro-2,2-dimethyl-1-propanamine, N-(1,1-difluoromethyl)morpholine, 1,1-difluoro-N,N-dimethylphenylmethanamine, N,N-diethyl-α,α-difluoro-3-pyridylmethanamine, N,N-diethyl-α,α-difluoro-2-pyridylmethanamine, diethyl-α,α-difluoro-(4-chlorophenyl)methanamine, N,N-diisopropyl-α,α-difluorophenylmethanamine, N,N-diethylyl-α,α-difluorophenylmethanamine, N,N-dimethyl-α,α-difluorophenylmethanamine, 2,2-difluoro-1,3-dimethylimidazolidine, 2,2-difluoro-1,3,3-trimethylpyrrolidine, [2,2,2]-2,2,5,5-tetrafluoro-3,3,6,6-tetramethyl-1,4-diazabicyclooctane and [2,2,2]-2,2,6,6-tetrafluoro-3,3,5,5-tetramethyl-1,4-diazabicyclooctane.

6. Process according to at least one of Claims 1 to 5, **characterized in that** the molar ratio of oxalyl fluoride to compounds of the formula (I) is 0.8:1 to 20:1.

7. Process according to at least one of Claims 1 to 6, **characterized in that** the reaction temperature is -50°C to 100°C.

8. Process according to at least one of Claims 1 to 7, **characterized in that** the reaction pressure is 0.8 to 20 bar.

9. Process according to at least one of Claims 1 to 8, **characterized in that** the reaction with
• at least one aprotic, tertiary amine which does not contain fluorine atoms in the α-position to the nitrogen and/or at least one N-heteroaromatic compound and
• hydrogen fluoride takes place in a further step.

10. Process according to Claim 9, **characterized in that** the molar ratio of aprotic tertiary amine and/or N-heteroaromatic compounds to compounds of the formula (I) is 0.1:1 to 20:1 and the molar ratio of hydrogen fluoride to aprotic tertiary amine is 0.2:1 to 10:1.

11. Process for preparing fluorine compounds from corresponding hydroxyl compounds and for preparing geminal difluorocompounds from the corresponding carbonyl compounds by using compounds which have been prepared according to at least one of Claims 1 to 10.

## Revendications

1. Procédé pour la préparation de composés de formule (I) 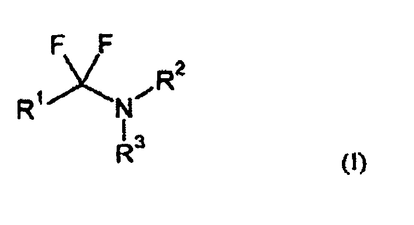 dans laquelle
R¹ représente un atome d'hydrogène, un groupe alkyle en C₁-C₁₂,
[ (alkylène en C₂-C₁₂) -O]ₙ (alkyle en C₁-C₁₂) où n = 1 à 5, aryle en C₃-C₁₄, arylalkyle en C₄-C₁₅ ou NR⁴R⁵, R⁴ et R⁵ représentant chacun, indépendamment l'un de l'autre, un groupe alkyle en C₁-C₈ ou bien NR⁴R⁵ représentant dans son ensemble un radical cyclique à 4-7 chaînons, ayant au total de 3 à 16 atomes de carbone et
R² et R³ représentent chacun, indépendamment l'un de l'autre, un groupe alkyle en C₁-C₁₂, aryle en C₃-C₁₄, arylalkyle en C₄-C₁₅ ou bien ensemble une partie d'un radical cyclique ayant au total de 3 à 16 atomes de carbone ou
R¹ et R² et/ou R³ représentent un radical cyclique ayant au total de 3 à 16 atomes de carbone,
**caractérisé en ce qu'**on fait réagir des composés de formule (II) 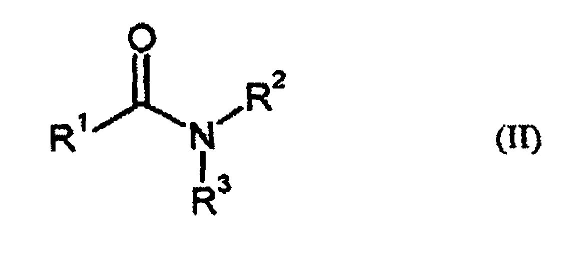 dans laquelle
R¹ , R² et R³ ont les significations données précédemment,
en présence de
• fluorure d'oxalyle ou
• fluorure d'oxalyle et difluorophosgène.

2. Procédé selon la revendication 1, **caractérisé en ce que** la réaction a lieu en présence d'un solvant organique.

3. Procédé selon au moins l'une des revendications 1 et 2, **caractérisé en ce que** R¹ représente un atome d'hydrogène, un groupe alkyle en C₁-C₁₂ ou aryle en C₃-C₆.

4. Procédé selon au moins l'une des revendications 1 à 3, **caractérisé en ce que** R² et R³ représentent chacun, indépendamment l'un de l'autre, un groupe alkyle en C₁-C₈, ou NR²R³ représente dans son ensemble un groupe N-morpholinyle, N-méthyl-1,4-pipérazin-N-yle, ou bien R¹CF₂R² représente dans son ensemble un groupe 2,2-difluoro-imidazolinyle, 2,2-difluoropyrrolidinyle, 2,2-difluoropipéridinyle ou [2.2.2]-2,2,5,5-tétrafluoro-1,4-diazabicyclo-octane ou [2,2,2]-2,2,6,6-tétrafluoro-1,4-diazabicyclo-octane, les radicaux nommés pouvant éventuellement être une ou plusieurs fois substitués par alkyle en C₁-C₄.

5. Procédé selon au moins l'une des revendications 1 à 4, **caractérisé en ce qu'**en tant que composés de formule (I) on prépare : la 1,1-difluorométhyl-N,N-diméthylamine, la 1,1-difluorométhyl-N,N-diéthylamine, la 1,1-difluorométhyl-N,N-diisopropylamine, la 1,1-difluoro-N,N-2-triméthyl-1-propanamine, la 1,1-difluoro-N,N-2,2-tétraméthyl-1-propanamine, la N,N-diéthyl-α,α-difluoro-2,2-diméthyl-1-propanamine, la N-(1,1-difluorométhyl)-morpholine, la 1,1-difluoro-N,N-diméthylphenylméthanamine, la N,N-diéthyl-α,α-difluoro-3-pyridylméthanamine, la N,N-diéthyl-α,α-difluoro-2-pyridylméthanamine, la diéthyl-α,α-difluoro-(4-chlorophényl)méthanamine, la N,N-diisopropyl-α,α-difluoro-phénylméthanamine, la N,N-diéthylyl-α,α-difluorophénylméthanamine, la N,N-diméthyl-α,α-difluoro-phénylméthanamine, la 2,2-difluoro-1,3-diméthylimidazolidine, la 2,2-difluoro-1,3,3-triméthylpyrrolidine, le [2,2,2]-2,2,5,5-tétrafluoro-3,3,6,6-tétraméthyl-1,4-diazabicyclo-octane et le [2.2.2]-2,2,6,6-tétrafluoro-3,3,5,5-tétraméthyl-1,4-diazabicyclooctane.

6. Procédé selon au moins l'une des revendications 1 à 5, **caractérisé en ce que** le rapport molaire du fluorure d'oxalyle aux composés de formule (I) va de 0,8:1 à 20:1.

7. Procédé selon au moins l'une des revendications 1 à 6, **caractérisé en ce que** la température de la réaction est dans la plage de -50°C à 100°C.

8. Procédé selon au moins l'une des revendications 1 à 7, **caractérisé en ce que** la pression dans la réaction est de 0,8 à 20 bars.

9. Procédé selon au moins l'une des revendications 1 à 8, **caractérisé en ce que** dans une autre étape on effectue la réaction avec
• au moins une amine tertiaire aprotique qui ne contient pas d'atomes de fluor en position α, par rapport à l'atome d'azote et/ou au moins un composé N-hétéroaromatique et
• de l'acide fluorhydrique.

10. Procédé selon la revendication 9, **caractérisé en ce que** le rapport molaire de l'amine tertiaire aprotique et/ou des composés N-hétéroaromatiques aux composés de formule (I) va de 0,1:1 à 20:1 et le rapport molaire de l'acide fluorhydrique à l'amine tertiaire aprotique va de 0,2:1 à 10:1.

11. Procédé pour la préparation de composés fluorés à partir des composés hydroxylés correspondants ainsi que pour la préparation de composés difluorés géminés à partir des composés carbonyle correspondants, au moyen de composés qui ont été préparés selon au moins l'une des revendications 1 à 10.
